# EUROPÄISCHE PATENTSCHRIFT

(11) **EP 3 672 932 B1**
(45) Veröffentlichungstag und Bekanntmachung des Hinweises auf die Patenterteilung: **24.01.2024**
(21) Anmeldenummer: 17768007.1
(22) Anmeldetag: 26.08.2017
(51) Int. Cl.: C07C 45/39, C07C 49/407

(54) **VERFAHREN ZUR HERSTELLUNG VON TERPENALDEHYDEN UND -KETONEN**
METHOD FOR PRODUCING TERPENE ALDEHYDES AND TERPENE KETONES
PROCÉDÉ DE PRÉPARATION D'ALDÉHYDES ET DE CÉTONES TERPÉNIQUES

(43) Veröffentlichungstag der Anmeldung: 01.07.2020
(73) Patentinhaber: Symrise AG, 37603 Holzminden (DE)
(72) Erfinder: KULIK, Anna, 13187 Berlin (DE); ECKELT, Reinhard, 18069 Rostock (DE); KÖCKRITZ, Angela, 12437 Berlin (DE); NEUBAUER, Katja, 18059 Rostock (DE)
(74) Vertreter: Daniels, Stefanie Lisa
(86) Internationale Anmeldenummer: PCT/EP2017/071478
(87) Internationale Veröffentlichungsnummer: WO 2019/042520

(56) Entgegenhaltungen:
- US-A1- 2004 204 597
- VINCIUS V COSTA ET AL: "Aerobic oxidation of monoterpenic alcohols catalyzed by ruthenium hydroxide supported on silica-coated magnetic nanoparticles", JOURNAL OF CATALYSIS, ACADEMIC PRESS, DULUTH, MN, US, Bd. 282, Nr. 1, 14. Juni 2011 (2011-06-14) , Seiten 209-214, XP028250350, ISSN: 0021-9517, DOI: 10.1016/J.JCAT.2011.06.014 [gefunden am 2011-06-18] in der Anmeldung erwähnt

## Beschreibung

### GEBIET DER ERFINDUNG

Die Erfindung befindet sich auf dem Gebiet der heterogenen Katalyse und betrifft ein Verfahren zur Herstellung von Terpenaldehyden und -ketonen aus Terpenalkoholen durch oxidative Dehydrierung.

### STAND DER TECHNIK

Terpenketone, allen voran Menthon, werden in der Parfümerie zur Erzeugung spezieller Minznoten in Parfümkompositionen verwendet. Die Reaktion kann durch folgendes Schema beispielhaft wiedergegeben werden:

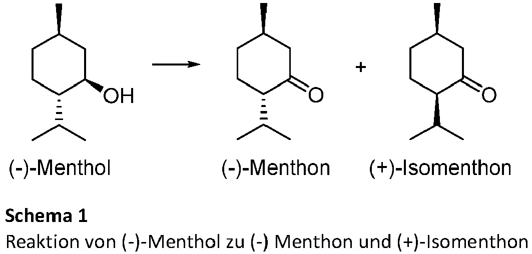

Aus dem Stand der Technik sind zahlreiche Verfahren bekannt, mit deren Hilfe man beispielsweise ausgehend von Menthol Menthon bzw. Gemische von Menthon und Isomenthon erhalten kann. Zu den eher veralteten Methoden gehören nichtkatalytische Verfahren, bei denen aus toxikologischer und ökologischer Sicht problematische Oxidationsmittel wie beispielsweise Dichromat, Permanganat oder Chlordioxid in stöchiometrischen Mengen zum Einsatz kommen [FROLOVA ET AL, Chem. Natural Comp. 44, S. 724 ff. (2008**)].**

Die aerobe katalytische Oxidation von sekundären Alkoholen zu Ketonen wurde bereits mit verschiedenen heterogenen Katalysatoren durchgeführt. Als Aktivmetalle sind beispielsweise Gold, Ruthenium, Palladium, Rhodium, Eisen, Kupfer oder Vanadium in geeigneten Oxidationsstufen und integriert in geeignete Materialien eingesetzt worden, die Reaktionen im Bereich der Feinchemikaliensynthesen erfolgten zumeist im diskontinuierlichen Modus in der Flüssigphase (vgl. RSC Green Chemistry Series (CARDONA ET AL, Band 28: Transition Metal Catalysis in Aerobic Alcohol Oxidation, The Royal Society of Chemistry, Cambridge, UK, 2015).

Ru(OH)ₓ bzw. RuOₓ xH₂O, welche Ruⁿ⁺-OH-Spezies enthalten, sind sehr aktive Katalysatoren für die aerobe oxidative Dehydrierung von Alkoholen. Die Voraussetzung für eine gute katalytische Aktivität ist das hochdisperse Aufbringen der aktiven Spezies auf einem geeigneten Trägermaterial, zum Beispiel Aluminiumoxid. Der so erhaltene geträgerte Katalysator wird bislang jedoch überwiegend in der Flüssigphase in Batchreaktoren eingesetzt [z.B. YAMAGUCHI ET AL, Angew. Chem 114 (2002) 4720**;** YAMAGUCHI ET AL, Chem. Eur. J. 9 (2003) 4353**;** YAMAGUCHI ET AL, Top. Catal. 57 (2014) 1196**;** PAGLIARO ET AL, Chem. Soc. Rev. 34 (2005) 837)].

Des Weiteren wurden Ru/MgO-La₂O₂-Mischoxide für die aerobe Oxidation aktivierter Alkohole in Toluol bei 80 °C eingesetzt [KANTAM ET AL, J. Mol. Catal. A: Chem. 359, 1 (2012**)].** Ru/CaO-ZrO₂ wurde ebenfalls untersucht, jedoch wurde kein cycloaliphatischer Alkohol als Substrat eingesetzt [YASUEDA ET AL, J. Mol. Catal. A Chem. 323, 7 (2010**)].** RuHAP (HAP = Hydroxyapatit) ist für die Oxidation von Benzylalkohol und 1-Phenylethanol als Katalysator geeignet [KIM ET AL, Bull. Korean Chem Soc. 34, 221 (2013**)].Mit** Ru/CeO₂ wurde Benzylalkohol zu Benzaldehyd oxidiert [WADA ET AL, Catal. Surv. Asia 15, 1 (2011**)].**

Die Zugabe weiterer oxidationsaktiver oxidischer Metallspezies wirkt sich günstig auf den Umsatz von primären Alkoholen und die Ausbeute an Aldehyden aus. So wurden Ru/TiO₂ oder Ru/ZrO₂, welche mit einem Zweitoxid dotiert waren, untersucht [KÖCKRITZ ET AL, J. Mol. Catal. A: Chem. 246, 85 (2006**)].** Eine weitere Verbesserung der Aktivität diese Katalysatortyps gelingt, wenn die oxidischen Metallspezies, insbesondere RuMnCe, nanopartikulär auf CeO₂ verteilt wurden [CHECINSKI ET AL, Appl. Catal. A: Gen. 366, 212 (2009**)].** Die Oxidation verschiedener primärer und sekundärer Alkohole zu Aldehyden und Ketonen gelang besonders gut in Gegenwart von RuMnMn-Spezies auf Hydrotalcit [EBITANI ET AL, Angew. Chem. Int. Ed. 44, 3423 (2005)].

Auch Aktivkohle ist als Trägermaterial für Rutheniumkatalysatoren für die Oxidation von primären und sekundären Alkoholen in organischen Lösungsmitteln geeignet, Terpene wurden jedoch nicht untersucht, und es erfolgte auch keine Angabe über die aktive Oxidationsstufe von Ruthenium [MORI ET AL, Chem. Comm. 5159 (2009**)].**

Ru(OH)ₓ/Al₂O₃ ist auch ein tauglicher Katalysator für einen kontinuierlichen Prozess in der Flüssigphase unter Verwendung eines Lösungsmittels, als Substrate wurden aber nur durch Aromaten oder Heteroaromaten aktivierte Alkohole verwendet **(**MANNEL ET AL, Org. Process Res. Dev. 18,1503 (2014**)]**

Insgesamt werden Terpene selten als Edukte für derartige Reaktionen beschrieben. Menthol ist zudem ein schwierig zu oxidierenderTerpenalkohol. Die alkoholische OH-Gruppe ist nicht aktiviert, sie befindet sich zudem in der Äquatorialposition. Aus dem Stand der Technik sind daher nur Reaktionen bekannt, die in der Flüssigphase bei Temperaturen deutlich unter 150 °C ablaufen. So gelang mit Ru(OH)ₓ/Fe₂O₃@SiO₂ die Oxidation von Menthol bei 10 Atmosphären Sauerstoffdruck nur mit 17% Umsatz und Ausbeute an Menthon bei 120 °C (COSTA ET AL, J. Catal. 282, 209 (2011**)].** Mit Ru/CeO₂ konnte eine Ausbeute an Menthon von nur 15% in Toluol bei 110 °C erreicht werden [VOCANSON ET AL, Synth. Comm. 28, 2577 (1998**)].**

Andere Batch-Verfahren zur Herstellung von Menthon benutzen Thymol als Edukt, welches einer partiellen Hydrierung an Pd-Katalysatoren im Autoklaven bei 175 °C unterworfen wird **(**US 3,124,614**),** oder Isopulegol wird an homogenen Ru-Dihydrido- bzw. Ir-Dihydrido-Phosphin-Katalysatoren über eine Hydrierungs-/Dehydrierungs-Sequenz zu Menthon umgesetzt **(**EP 2706054 A1**).**

Das Dokument US 2004/204597 A1 offenbart ein Verfahren zur Herstellung von Ketonen, Aldehyden, Carbonsäuren und dergleichen durch Oxidation von Alkoholen mit molekularem Sauerstoff in Gegenwart eines Ruthenium-Trägerkatalysators.

Die kontinuierliche nichtoxidative Dehydrierung von Menthol zu Menthon gelingt in der Gasphase an Cu/Zn- oder Cu-Chromit-Katalysatoren **(**DE 4236111 A1**)** oder an ZnO/CaCO₃-Katalysatoren **(**WO 2005 085160 A1**),** allerdings wird dabei entweder bevorzugt bei vermindertem Druck gearbeitet, was eine apparative Aufrüstung erfordert, oder es sind Temperaturen deutlich über 300 °C erforderlich, welche die Bildung von unerwünschten Nebenprodukten begünstigen.

Es existiert kein Verfahren der kontinuierlichen oxidativen Dehydrierung zur Synthese von Terpenaldehyden bzw. Terpenketonen im Allgemeinen und Menthon im Besonderen in der Gasphase. Die konkrete Aufgabe der vorliegenden Erfindung hat daher darin bestanden, dem abzuhelfen und ein Verfahren zur Verfügung zu stellen, das sich dadurch auszeichnet, dass gleichzeitig
- gute Ausbeuten an einem Terpenaldehyd bzw. -keton im Allgemeinen und Menthon/Isomenthon-Gemischen im Besonderen erhalten werden,
- keine Racemisierung an chiralen Zentren erfolgt,
- kein Lösungsmittel verwendet und abgetrennt werden muss,
- keine Abtrennung des Katalysators erforderlich ist,
- kein Arbeiten unter vermindertem Druck notwendig ist,
- der verwendete Katalysator ein Arbeiten bei solchen Temperaturen erlaubt, bei denen eine Nebenproduktbildung relativ gering ist,
- in der Gasphase kein Leaching der Aktivmetalle in das Produkt stattfindet, und
- eine Qualität erhalten wird, die für die Parfümindustrie geeignet ist, d.h. das Reaktionsprodukt soll keine olfaktorisch störenden Nebenprodukte enthalten.

### BESCHREIBUNG DER ERFINDUNG

Der Gegenstand der Erfindung betrifft Verfahren zur Herstellung von Terpenaldehyden und -ketonen durch oxidative Dehydrierung der entsprechenden Terpenalkohole, umfassend oder bestehend aus den folgenden Schritten:
(a) Bereitstellung von Terpenalkoholen oder Terpenalkohol-haltigen Edukten;
(b) Inkontaktbringen der Ausgangsstoffe aus Schritt (a) mit einem heterogenen Ruthenium-Katalysator;
(c) Aufheizen der Mischung aus Schritt (b) in Gegenwart von Sauerstoff auf mindestens 150°C; sowie gegebenenfalls
(d) Abtrennung der Terpenaldehyde bzw. Terpenketone vom erhaltenen Reaktionsgemisch,
wobei die Reaktion in einem Reaktor durchführt wird, der kontinuierlich von einem Gasstrom durchströmt wird, wobei dieser Gasstrom beim Eintritt in den Reaktor mindestens einen Terpenalkohol sowie ein sauerstoffhaltiges Gas sowie gegebenenfalls ein Inertgas umfasst.

Überraschenderweise wurde gefunden, dass das eingangs geschilderte Anforderungsprofil durch das erfindungsgemäße Verfahren voll umfänglich erfüllt wird: Die Terpenaldehyde bzw. -ketone werden in hohen Ausbeuten und mit hohen Selektivitäten erhalten, die deutlich über denen liegen, die bisher gemäß Stand der Technik erreichbar waren. Weder musste Lösungsmittel mitverwendet werden, noch bestand das Erfordernis, den Katalysator aufwendig abzutrennen; das Verfahren erfordert auch keinen verminderten Druck. Der einzuhaltende Temperaturbereich ist so niedrig, dass es zu keiner nennenswerten Nebenproduktbildung kommt, so dass Qualitäten erhalten werden, die die strengen Anforderungen in der Parfümindustrie erfüllen.

### TERPENALKOHOLE

Grundsätzlich ist das erfindungsgemäße Verfahren auf alle Terpenalkohole anwendbar. Beispiele für geeignete Terpenalkohole umfassen Borneole und Fenchole, sowie insbesondere Carveol, Citronellol, Cuminalkohol, Dihydrocarveol, Farnesol, Geraniol, Nerol, Perillaalkohol, Phytol und Rhodinol sowie deren Gemische. Vorzugsweise wird im Sinne des erfindungsgemäßen Verfahrens Menthol eingesetzt, das zu Menthon bzw. einer Mischung aus Menthon und Isomenthon umgesetzt wird.

Es ist dabei möglich, die Terpenalkohole als Einzelstoffe einzusetzen. Ebenso können Mischungen verschiedener Terpenalkohole umgesetzt werden oder aber Naturstoffextrakte, die einen Gehalt an diesen Terpenalkoholen neben anderen Komponenten aufweisen.

### KATALYSATOREN

Gemäß vorliegender Erfindung werden heterogene Ruthenium-Katalysatoren eingesetzt, die vorzugsweise eine Oxidationsstufe größer Null aufweisen. Diese Katalysatoren können zudem weitere katalytisch aktive Spezies, insbesondere andere Metalloxide aufweisen. Diese können ausgewählt sein aus der Gruppe, die gebildet wird von der Ceroxiden, Kupferoxiden, Eisenoxiden, Manganoxiden, Kobaltoxiden, Molybdänoxiden, Silberoxiden und deren Mischungen. Besonders bevorzugte Beispiele stellen die folgenden Oxide dar: CeO₂, CuO, Cu₂O, Fe₂O₃, Fe₃O₄, MnO, MnO₂, Mn₂O₃, Mn₃O₄, Co₃O₄, CoO, MoOs, AgO, Ag₂O und deren Mischungen. Die Dotierung, beispielsweise in Mengen von etwa 10 bis etwa 50 Gew.-% bezogen auf die Rutheniummenge, führt zu einer signifikanten Steigerung der Katalysatoraktivität.

Im Sinne der vorliegenden Erfindung werden insbesondere solche Katalysatoren eingesetzt, bei dem die katalytisch aktiven Spezies auf einem Trägermaterial aufgebracht sind. Dabei ist es vorteilhaft, diese oxidierten Metallspezies - sowohl auf Ruthenium als auch auf den anderen Metallen basierend - sehr feinverteilt auf den Träger aufzubringen. Besonders bevorzugt ist die Ausführung der Erfindung, wenn der Katalysatorträger selbst aus einem für Oxidationen katalytisch aktiven Material besteht. Die Herstellung des aktiven Katalysators erfolgt über nasschemische Methoden wie Imprägnierung, Imprägnierung bis zur eintretenden Feuchte, Auffällung oder Co-Fällung. Die einzubringenden Metalle werden in Form geeigneter Präkursoren, z.B. als Metallchloride, Metallnitrate oder Metallacetate in der Katalysatorsynthese eingesetzt. Um erfindungsgemäß in einem kontinuierlichen Reaktor verwendet werden zu können, wird anschließend die rohe Katalysatormasse einer Formgebung wie Verstrangung oder Pelletierung sowie einer thermische Behandlung unterworfen. Bei den Trägern handelt es sich vorzugsweise um oxidische Materialien, die ausgewählt sind aus der Gruppe, die gebildet wird von Ceroxid, Aluminiumoxid, Zirkonoxid, Titanoxid, Siliziumoxid, Kieselsäure, Eisenoxid, Manganoxid, Nioboxid, Aluminosilikat, Hydrotalcit, Hydroxyapatit, sowie deren Mischungen. Alternativ kann auch Aktivkohle als Träger dienen.

Die Konzentration von Ruthenium bzw. der Mischung aus Ruthenium und weiteren Metallen in ihrer jeweiligen oxidischen Form auf dem Trägermaterial kann zwischen etwa 0,1 und etwa 10 Gew.-% und vorzugsweise zwischen etwa 0,5 und etwa 5 Gew.-% liegen.

### VERFAHREN

Erfindungsgemäß benutzt man für die oxidative Dehydrierung von Menthol kontinuierliche Rohrreaktoren. Dabei kann der Katalysator in einem Festbett oder Wirbelbett in Kontakt mit dem Edukt treten. Die erfindungsgemäße Ausführung der Reaktionsanlage beinhaltet auch Rohrbündelreaktoren.

Der im Reaktor eingebrachte Katalysator wird von gasförmigen Reaktanden durchströmt. Diese Reaktanden bestehen aus gasförmigem Menthol und einem sauerstoffhaltigen Gas. Das sauerstoffhaltige Gas wird durch Massendurchflussregler in geeigneten Konzentrationen zugeführt. Die Konzentration von Sauerstoff in dem sauerstoffhaltigen Gas beläuft sich mindestens auf 0,1%. Die Zuführung des Terpenalkohols erfolgt über eine Pumpe, die Überführung in die Gasphase wird durch technikübliche Verdampferkonstruktionen vollzogen.

Das erfindungsgemäße Verfahren erfordert eine Mindesttemperatur von 150 °C, um sicherzustellen, dass die Reaktion in der Gasphase abläuft, da dies für Umsätze und Selektivität kritisch ist. Ansonsten beträgt der bevorzugte Temperaturbereich 150 bis etwa 350 °C, vorzugsweise etwa 180 bis etwa 320 °C und insbesondere 250 bis etwa 300 °C.

Durch die Einstellung von optimaler Temperatur und Verweilzeit bzw. GHSV (gas hourly space velocity) kann die Ausbeute und Selektivität beispielsweise des gewünschten Menthon/Isomenthon-Gemisches maximiert werden. Darin ist ein Vorteil dieses erfindungsgemäßen kontinuierlichen Verfahrens zu sehen.

Die Reaktion wird in einem Reaktor durchzuführt, der kontinuierlich von einem Gasstrom durchströmt wird, wobei dieser Gasstrom beim Eintritt in den Reaktor den Terpenalkohol, vorzugsweise das Menthol sowie ein sauerstoffhaltiges Gas sowie gegebenenfalls ein Inertgas umfasst. Das sauerstoffhaltige Gas enthält dabei vorzugsweise mindestens 0,1 Vol.-% Sauerstoff, bezogen auf das Gesamtvolumen des sauerstoffhaltigen Gases, bestimmt bei 20°C und 1013,25 hPa.

Eine weitere bevorzugte Ausführungsform des erfindungsgemäßen Verfahrens besteht ferner darin, dass
(ii) die Strömungsrate des Gasstroms bezogen auf das Volumen des heterogenen Katalysators (gas hourly space velocity GHSV) etwa 100 h⁻¹ bis etwa 5.000 h⁻¹, vorzugsweise etwa 200 h⁻¹ bis etwa 1.000 h⁻¹ beträgt und/oder
(ii) die Konzentration an Terpenalkohol in dem in den Reaktor eintretenden Gasstrom etwa 1 Mol-% bis etwa 15 Mol-%, vorzugsweise etwa 3 Mol-% bis etwa 10 Mol-% beträgt.

Geeignete Verweilzeiten bewegen sich dabei im Bereich von τ = 1-20 s. Am Ausgang des Reaktors wird das gasförmige Produktgemisch kondensiert und einer gas/flüssig-Trennung unterworfen. Das flüssige Produktgemisch kann anschließend destillativ aufgereinigt werden.

Das Verfahren kann dabei sowohl batchweise, als auch vorzugsweise kontinuierlich durchgeführt werden.

### BEISPIELE 1 BIS 9

### Allgemeine Beschreibung der verwendeten Apparatur und der Reaktionsführung

Die verwendete kontinuierliche Rohrreaktor-Apparatur setzte sich zusammen aus einem Sättiger, Thermostat, Gasleitungen, Gasdosierung und -regelung mit digitalen MFCs, einem Rohrreaktor mit Ofen, einem im Ölbad temperierten Probenauffanggefäß (befüllt mit 6 mL o-Xylol) für Hochsieder sowie einem in einem Kryostaten gekühlten Probenauffanggefäß für Niedrigsieder und mehrerer Heizungen mit Steuer- sowie Thermoelementen.

Zur Durchführung der Versuche wurde zunächst der Edelstahlrohrreaktor mit dem zuvor granuliertem Katalysator (3 mL) befüllt. Der mit Menthol befüllte Sättiger wurde anschließend in die Thermostatlösung, die auf die gewünschte Temperatur (meist 120°C) erhitzt wurde, getaucht. Mit Ausnahme der Gasleitung zum Sättiger wurde die Temperatur aller weiteren Leitungen durch Heizungen (Heizbänder) mit Thermoelementen und Steuergeräten auf 200°C eingestellt und konstant gehalten. Während der Aufheizphase wurde die komplette Versuchsapparatur über eine am Sättiger vorbeiführende Gasleitung mit N₂ gespült. In den auf 120°C temperierten Sättiger wurde anschließend ein Gasgemisch aus 5 Vol.-% O₂/Ar (30 mL/min) geleitet, nachdem die N₂-Spülgaszufuhr geschlossen wurde. Durch das Öffnen des Gasventils vom Sättiger zum Reaktor wurde die Reaktion gestartet. Um das Auskristallisieren des nicht umgesetzten Menthols (Smp.: 41-45°C) am Reaktorausgang zu verhindern, wurde die Gasleitung vom Reaktorausgang zum Auffanggefäß zusätzlich auf 100°C beheizt. Darüber hinaus wurde das Probengefäß mit 6 mL o-Xylol als Auffanglösung mit Hilfe eines auf 60°C temperierten Ölbades während der gesamten Reaktionszeit erhitzt. Eine Kühlfalle zum Auffangen eventuell entstandener flüchtigerer Produkte befand sich direkt hinter der eigentlichen Probenentnahmestelle. Nachdem die Reaktion beendet war, wurde das Ventil vom Sättiger zum Reaktor geschlossen und N₂ zum Spülen der Anlage eingeleitet. Anschließend wurden alle weiteren Steuerelemente zur Gasdosierung und Temperaturregelung ausgeschaltet.

Zur Ermittlung des Mentholverbrauchs während der Reaktion wurde der Sättiger nach jedem Versuch ausgebaut und ausgewogen. Die Gesamtreaktionszeit pro Temperatur betrug dabei stets 3 h, wobei eine stündliche Probenentnahme erfolgte. Dabei wurden die Versuche unter den folgenden Bedingungen durchgeführt:
- 3 mL Katalysator,
- 5 mol.-% Menthol im Gasstrom,
- 30 mL/min 5 Vol.-% O₂/Ar,

Eingesetzte Katalysatoren:
- [a]:Ru/C,
- [b]: 0,2 %Ru/CeO₂,
- [c]: 0,2-1,0/1,2/1,6% RuMnCe/CeO₂,
wobei die Katalysatoren [a] und [b] Handelsprodukte darstellen und Katalysator [c] nach folgender Standardvorschrift erhalten wurde:

Zu einer Suspension aus 4,46 g CeO₂ in 100 mL deionisiertem Wasser wurde eine Lösung aus 23,4 mg RuCl₃·H₂O, 260,6 mg Mn(NO₃)₂·4H₂O und 225,4 mg Ce(NO₃)₃·6H₂O in 20 mL Wasser gegeben. Anschließend wurde mit Hilfe einer Spritzenpumpe eine Lösung aus 415,2 mg NaOH und 314,4 mg Na₂CO₃ in 8 mL Wasser unter Rühren der Suspension innerhalb einer Zeitspanne von 5 h kontinuierlich zugetropft. Nach beendeter Zugabe wurde die Reaktionssuspension auf 65°C erhitzt und für 18 h gerührt. Der Feststoff wurde durch Zentrifugieren abgetrennt, dreimal mit je 20 mL Wasser gewaschen und über Nacht bei 90°C im Trockenschrank getrocknet. Die Aktivkomponenten Ru, Mn und Ce liegen auf dem Träger als Oxide und/oder Hydroxide vor.

Beobachtete Umsätze und Ausbeuten sind in der nachfolgenden **Tabelle 1** dargestellt.

**Tabelle 1**

| Umsätze und Ausbeuten | | | | | | | | |
|---|---|---|---|---|---|---|---|---|
| **Bsp.** | **Ru-Gehalt** | **T [°C]** | **X_{OL} [mol-%]** | **Y_{ON} [mol-%]** | **Y_{ISO} [mol-%]** | **Y_{NP} [mol-%]** | **S_{ON} [%]** | **S_{ISO} [%]** |
| 1 | 5^{[a]} | 300 | 99 | 4 | 2 | 59 | 4 | 2 |
| 2 | 0,2^{[b]} | 300 | 92 | 31 | 22 | 11 | 34 | 24 |
| 3 | 0,2^{[b]} | 350 | 93 | 31 | 23 | 10 | 33 | 25 |
| 4 | 0,2^{[c]} | 300 | 52 | 20 | 17 | 4 | 39 | 32 |
| 5 | 0,2^{[c]} | 350 | 68 | 27 | 23 | 6 | 40 | 34 |
| 6 | 0,5^{[c]} | 300 | 94 | 38 | 26 | 5 | 40 | 28 |
| 7 | 0,5^{[c]} | 350 | 96 | 37 | 27 | 5 | 38 | 28 |
| 8 | 1,0^{[c]} | 300 | 92 | 40 | 28 | 6 | 43 | 31 |
| 9 | 1,0^{[c]} | 350 | 93 | 40 | 29 | 9 | 42 | 32 |

| | | | | | | | | |
|---|---|---|---|---|---|---|---|---|
| Dabei bedeuten: X_{OL} = Umsatz Menthol in Mol-% Y_{ON} = Ausbeute Menthon in Mol-% Y_{ISO} = Ausbeute Isomenthon in Mol% Y_{NP} = Ausbeute Nebenprodukte in Mol-% S_{ON} =Selektivität zu Menthon S_{ISO} = Selektivität zu Isomenthon | | | | | | | | |

## Patentansprüche

1. Verfahren zur Herstellung von Terpenaldehyden und -ketonen durch oxidative Dehydrierung der entsprechenden Terpenalkohole, umfassend oder bestehend aus den folgenden Schritten:
(a) Bereitstellung von Terpenalkoholen oder Terpenalkohol-haltigen Edukten;
(b) Inkontaktbringen der Ausgangsstoffe aus Schritt (a) mit einem heterogenen Ruthenium-Katalysator;
(c) Aufheizen der Mischung aus Schritt (b) in Gegenwart von Sauerstoff auf mindestens 150°C; sowie gegebenenfalls
(d) Abtrennung der Terpenaldehyde bzw. Terpenketone vom erhaltenen Reaktions-gemisch,
wobei man die Reaktion in einem Reaktor durchführt, der kontinuierlich von einem Gasstrom durchströmt wird, wobei dieser Gasstrom beim Eintritt in den Reaktor mindestens einen Terpenalkohol sowie ein sauerstoffhaltiges Gas sowie gegebenenfalls ein Inertgas umfasst.

2. Verfahren nach Anspruch 1, **dadurch gekennzeichnet, dass** man als Terpenalkohol Menthol einsetzt.

3. Verfahren nach den Ansprüchen 1 und/oder 2, **dadurch gekennzeichnet, dass** man einen heterogenen Ruthenium-Katalysator einsetzt, der weitere Metalloxide enthält.

4. Verfahren nach Anspruch 3, **dadurch gekennzeichnet, dass** man Metalloxide einsetzt, die ausgewählt sind aus der Gruppe, die gebildet wird von den Ceroxiden, Kupferoxiden, Eisenoxiden, Manganoxiden, Cobaltoxiden, Molybdänoxiden, Silberoxiden und deren Mischungen.

5. Verfahren nach Anspruch 3 und/oder 4, **dadurch gekennzeichnet, dass** man Metalloxide einsetzt, die ausgewählt sind aus der Gruppe, die gebildet wird von CeO₂, CuO, CuzO, Fe₂O₃, Fe₃O₄, MnO, MnOz, Mn₂O₃, Mn₃O₄, Co₃O₄, CoO, MoO₃, AgO, Ag₂O und deren Mischungen.

6. Verfahren nach mindestens einem der Ansprüche 1 bis 5, **dadurch gekennzeichnet, dass** man einen heterogenen Ruthenium-Katalysator einsetzt, bei dem die katalytisch aktiven Spezies auf einem Trägermaterial aufgebracht sind.

7. Verfahren nach Anspruch 6, **dadurch gekennzeichnet, dass** man ein Trägermaterial einsetzt, das ebenfalls katalytisch aktiv ist.

8. Verfahren nach den Ansprüchen 6 und/oder 7, **dadurch gekennzeichnet, dass** man ein oxidisches Trägermaterial einsetzt.

9. Verfahren nach mindestens einem der Ansprüche 6 bis 8, **dadurch gekennzeichnet, dass** man ein Trägermaterial einsetzt, das ausgewählt ist aus der Gruppe, die gebildet wird von Ceroxid, Aluminiumoxid, Zirkonoxid, Titanoxid, Siliziumoxid, Kieselsäure, Eisenoxid, Manganoxid, Nioboxid, Aluminosilikat, Hydrotalcit, Hydroxyapatit, sowie deren Mi-schungen.

10. Verfahren nach Anspruch 6, **dadurch gekennzeichnet, dass** man als Trägermaterial Aktivkohle einsetzt.

11. Verfahren nach einem der vorherigen Ansprüche, **dadurch gekennzeichnet, dass** man einen heterogenen Ruthenium-Katalysator einsetzt, bei dem die Konzentration von Ruthenium auf dem Trägermaterial zwischen etwa 0,1 und etwa 10 Gew.-% und vorzugs-weise zwischen etwa 0,5 und etwa 5 Gew.-% beträgt.

12. Verfahren nach mindestens einem der Ansprüche 1 bis 11, **dadurch gekennzeichnet, dass** man die Reaktion bei einer Temperatur im Bereich von 150 bis etwa 350 °C, vorzugsweise im Bereich von etwa 180 bis etwa 320 °C und insbesondere im Bereich von etwa 250 bis etwa 300 °C durchführt.

13. Verfahren nach mindestens einem der Ansprüche 1 bis 12, **dadurch gekennzeichnet, dass** das sauerstoffhaltige Gas mindestens 0,1 Vol.-% Sauerstoff, bezogen auf das Gesamtvolumen des sauerstoffhaltigen Gases, bestimmt bei 20°C und 1013,25 hPa, enthält.

14. Verfahren nach mindestens einem der Ansprüche 1 bis 12, **dadurch gekennzeichnet, dass**
(i) die Strömungsrate des Gasstroms bezogen auf das Volumen des heterogenen Katalysa-tors (gas hourly space velocity GHSV) etwa 100 h⁻¹ bis etwa 5.000 h⁻¹, vorzugsweise etwa 200 h⁻¹ bis etwa 1.000 h⁻¹ beträgt und/oder
(ii) die Konzentration an Terpenalkohol in dem in den Reaktor eintretenden Gasstrom etwa 1 Mol-% bis etwa 15 Mol-%, vorzugsweise etwa 3 Mol-% bis etwa 10 Mol-% beträgt.

## Claims

1. A process for the preparation of terpene aldehydes and ketones by oxidative dehydrogenation of the corresponding terpene alcohols, comprising or consisting of the following steps:
(a) providing terpene alcohols or terpene alcohol-containing reactants;
(b) contacting the starting materials from step (a) with a heterogeneous ruthenium catalyst;
(c) heating the mixture from step (b) in the presence of oxygen to at least 150°C; and optionally
(d) separating the terpene aldehydes or terpene ketones from the resulting reaction mixture,
wherein the reaction is carried out in a reactor through which a gas stream flows continuously, said gas stream comprising at least one terpene alcohol and an oxygen-containing gas and optionally an inert gas upon entry into the reactor.

2. The process according to Claim 1, **characterized in that** menthol is used as terpene alcohol.

3. The process according to Claims 1 and/or 2, **characterized in that** a heterogeneous ruthenium catalyst is used which contains further metal oxides.

4. The process according to Claim 3, **characterized in that** metal oxides selected from the group formed by cerium oxides, copper oxides, iron oxides, manganese oxides, cobalt oxides, molybdenum oxides, silver oxides and mixtures thereof are used.

5. The process according to Claim 3 and/or 4, **characterized in that** metal oxides are used which are selected from the group formed by CeO₂, CuO, Cu₂O, Fe₂O₃, Fe₃O₄, MnO, MnO₂, Mn₂O₃, Mn₃O₄, Co₃O₄, CoO, MoOs, AgO, Ag₂O and mixtures thereof.

6. The process according to at least one of Claims 1 to 5, wherein a heterogeneous ruthenium catalyst is used in which the catalytically active species are supported on a carrier material.

7. The process according to Claim 6, **characterized in that** a support material is used which is also catalytically active.

8. The process according to Claims 6 and/or 7, **characterized in that** an oxidic support material is used.

9. The process according to at least one of Claims 6 to 8, **characterized in that** a support material is used which is selected from the group formed by cerium oxide, aluminum oxide, zirconium oxide, titanium oxide, silicon oxide, silicic acid, iron oxide, manganese oxide, niobium oxide, aluminosilicate, hydrotalcite, hydroxyapatite, and mixtures thereof.

10. The process according to Claim 6, **characterized in that** activated carbon is used as the carrier material.

11. The process according to one of the preceding claims 1 to 10, **characterized in that** a heterogeneous ruthenium catalyst is used, in which the concentration of ruthenium on the support material is between about 0.1 and about 10% by weight and preferably between about 0.5 and about 5% by weight.

12. The process according to at least one of Claims 1 to 11, **characterized in that** the reaction is carried out at a temperature in the range from 150 to about 350 °C, preferably in the range from about 180 to about 320 °C and in particular in the range from about 250 to about 300 °C.

13. The process according to at least one of Claims 1 to 12, **characterized in that** the oxygen-containing gas contains at least 0.1% by volume of oxygen, based on the total volume of the oxygen-containing gas, determined at 20°C and 1013.25 hPa.

14. The process according to at least one of Claims 1 to 12, **characterized in that**
(i) the flow rate of the gas stream relative to the volume of the heterogeneous catalyst (gas hourly space velocity GHSV) is about 100 h⁻¹ to about 5,000 h⁻¹, preferably about 200 h⁻¹ to about 1,000 h⁻¹, and/or
(ii) the concentration of terpene alcohol in the gas stream entering the reactor is about 1 mol% to about 15 mol%, preferably about 3 mol% to about 10 mol%.

## Revendications

1. Procédé de préparation d'aldéhydes et de cétones terpéniques par déshydrogénation oxydante des alcools terpéniques correspondants, comprenant ou consistant en les étapes suivantes :
(a) fourniture d'alcools terpéniques ou de produits de départ contenant des alcools terpéniques ;
(b) mise en contact des produits de départ de l'étape (a) avec un catalyseur au ruthénium hétérogène ;
(c) chauffage du mélange de l'étape (b) en présence d'oxygène à au moins 150°C ; et éventuellement
(d) la séparation des aldéhydes terpéniques ou des cétones terpéniques du mélange réactionnel obtenu,
dans lequel on effectue la réaction dans un réacteur traversé en continu par un flux gazeux, ce flux gazeux comprenant à l'entrée du réacteur au moins un alcool terpénique ainsi qu'un gaz contenant de l'oxygène et éventuellement un gaz inerte.

2. Procédé selon la revendication 1, **caractérisé en ce que** l'on utilise du menthol comme alcool terpénique.

3. Procédé selon les revendications 1 et/ou 2, **caractérisé en ce que** l'on utilise un catalyseur hétérogène au ruthénium, qui contient d'autres oxydes métalliques.

4. Procédé selon la revendication 3, **caractérisé en ce que** l'on utilise des oxydes métalliques choisis dans le groupe formé par les oxydes de cérium, les oxydes de cuivre, les oxydes de fer, les oxydes de manganèse, les oxydes de cobalt, les oxydes de molybdène, les oxydes d'argent et leurs mélanges.

5. Procédé selon la revendication 3 et/ou 4, **caractérisé en ce qu'**on utilise des oxydes métalliques choisis dans le groupe formé par CeO₂, CuO, Cu₂O, Fe₂O₃, Fe₃O₄, MnO, MnO₂, Mn₂O₃, Mn₃O₄, Co₃O₄, CoO, MoOs, AgO, Ag₂O et leurs mélanges.

6. Procédé selon au moins l'une des revendications 1 à 5, **caractérisé en ce que** l'on met en oeuvre un catalyseur hétérogène au ruthénium dans lequel les espèces catalytiquement actives sont déposées sur un matériau support.

7. Procédé selon la revendication 6, **caractérisé en ce que** l'on utilise un matériau support qui est également catalytiquement actif.

8. Procédé selon les revendications 6 et/ou 7, **caractérisé en ce que** l'on utilise un matériau support de type oxyde.

9. Procédé selon au moins l'une des revendications 6 à 8, **caractérisé en ce que** l'on utilise un matériau support choisi dans le groupe formé par l'oxyde de cérium, l'oxyde d'aluminium, l'oxyde de zirconium, l'oxyde de titane, l'oxyde de silicium, la silice, l'oxyde de fer, l'oxyde de manganèse, l'oxyde de niobium, l'aluminosilicate, l'hydrotalcite, l'hydroxyapatite, ainsi que leurs méta-solutions.

10. Procédé selon la revendication 6, **caractérisé en ce que** l'on utilise du charbon actif comme matériau support.

11. Procédé selon l'une quelconque des revendications précédentes, **caractérisé en ce que** l'on utilise un catalyseur hétérogène au ruthénium dans lequel la concentration en ruthénium sur le support est comprise entre environ 0,1 et environ 10% en poids et de préférence entre environ 0,5 et environ 5% en poids.

12. Procédé selon au moins l'une des revendications 1 à 11, **caractérisé en ce que** l'on effectue la réaction à une température comprise entre 150 et environ 350°C, de préférence entre environ 180 et environ 320°C, et en particulier entre environ 250 et environ 300°C.

13. Procédé selon au moins l'une des revendications 1 à 12, **caractérisé en ce que** le gaz contenant de l'oxygène contient au moins 0,1 % en volume d'oxygène par rapport au volume total du gaz contenant de l'oxygène, déterminé à 20°C et 1013,25 hPa.

14. Procédé selon au moins l'une des revendications 1 à 12, **caractérisé en ce que**
(i) le débit du flux de gaz par rapport au volume du catalyseur hétérogène (gas hourly space velocity GHSV) est d'environ 100 h⁻¹ à environ 5000 h⁻¹, de préférence d'environ 200 h⁻¹ à environ 1000 h⁻¹ et/ou
(ii) la concentration d'alcool terpénique dans le courant gazeux entrant dans le réacteur est d'environ 1 % en moles à environ 15 % en moles, de préférence d'environ 3 % en moles à environ 10 % en moles.
